# EUROPEAN PATENT SPECIFICATION

(11) **EP 3 910 514 B1**
(45) Date of publication and mention of the grant of the patent: **23.07.2025**
(21) Application number: 21172985.0
(22) Date of filing: 10.05.2021
(51) Int. Cl.: G06F 21/62, G06F 21/64, H04L 9/00, H04L 9/32, H04L 9/40, G16H 10/60, G16H 50/80

(54) **BLOCKCHAIN-BASED HEALTH MONITORING SYSTEM**
AUF BLOCKCHAIN BASIERENDES GESUNDHEITSÜBERWACHUNGSSYSTEM
SYSTÈME DE SURVEILLANCE DE LA SANTÉ À BASE DE CHAÎNE DE BLOCS

(30) Priority: 13.05.2020 IT 202000010861
(43) Date of publication of application: 17.11.2021
(73) Proprietor: Ali Group S.r.l., 20063 Cernusco sul Naviglio (MI) (IT)
(72) Inventor: COCCHI, Andrea, 40012 CALDERARA DI RENO (BOLOGNA) (IT); LAZZARINI, Roberto, 42100 REGGIO EMILIA (IT); TASSI, Federico, 40122 BOLOGNA (IT); STEFANELLI, Cesare, 40123 BOLOGNA (IT); GIANNELLI, Carlo, 40123 BOLOGNA (IT)
(74) Representative: Milli, Simone

(56) References cited:
- US-A1- 2017 091 397
- US-A1- 2018 308 566
- CISNEROS BENJAMIN ET AL: "CoviReader: Using IOTA and QR Code Technology to Control Epidemic Diseases across the US", 2021 IEEE 11TH ANNUAL COMPUTING AND COMMUNICATION WORKSHOP AND CONFERENCE (CCWC), IEEE, 27 January 2021 (2021-01-27), pages 610 - 618, XP033887603, DOI: 10.1109/CCWC51732.2021.9376093

## Description

This invention relates to a health supervision system, for example, a health supervision system capable of recording the health situations of a plurality of users.

The spread of Covid-19 has led to the adoption of restrictive measures which have resulted in a drastic reduction in trade and which, in many cases, have brought production activities to a halt. Concluding this period of restrictions will necessarily require highly precautionary measures to prevent new clusters of infection. Until such time as vaccines become available, the risk not only of the resurgence of internal clusters but also of importing infections from abroad remains high. In this context, work places are considered potential sources of biological hazard.

In the current context, therefore, the many new challenges that are emerging involve minimizing and monitoring new infections or improving the safety of people at work.

Monitoring the state of health of users may be linked to one or more of the following issues: ensuring data immutability, allowing access to information only by authorized persons (for example, health and/or judicial authorities) and guaranteeing the privacy and the right to be forgotten of the persons monitored.

Document US2017091397A1 shows a blockchain configured device-driven disintermediated distributed system for facilitating multi-faceted communication over a network. The system includes: entities connected with a communications network, each of the entities and associated devices and sensors and networks serve as a source of data records; a blockchain configured data bank accessible by each of the plurality of entities based on rules and preferences of the entities upon authorization by the blockchain configured data bank, the blockchain configured data bank including a processing component for executing stored instructions to process the data records of the entities over the communications network; a blockchain configured component communicatively coupled to the blockchain configured data bank and adapted to be accessible by each of the plurality of entities; a validation device including a facial expression-based validation device and a geo-tagging-based validation device.

Document US2018308566A1 discloses a system and method that facilitates the automated replication of electronic medical record information between a patient and a health-care provider (HCP) and provides support and security, such as by geographically distributed data fragmentation, for mobile platforms and web-based platforms and sophisticated mechanisms for the transmission of data between these systems. The system uses: a cloud-based infrastructure that includes databases, mathematical models, and configuration information; a patient's electronic health record system providing personal data around a patient's individual personal electronic medical record (PEMR); and a server used to coordinate and authenticate the replication of data between the cloud-based infrastructure, the PEMR, and the EMR/EHR system of the HCP.

This invention therefore has for an aim to meet the above mentioned needs by providing a health supervision system as described in this disclosure, capable of monitoring the health situation of the public with a view to limiting the spread of the virus.

Also an aim of this invention is to allow recorded data to remain immutable while at the same time guaranteeing the users' right to be forgotten.

A further aim of this invention is to ensure the traceability of the tests performed, as set out in Resolution No. 350, dated 16/04/2020, passed by the Emilia Romagna regional council.

The technical features of the disclosure, with reference to the above aims, are clearly described in the claims below and its advantages are apparent from the detailed description which follows, with reference to the accompanying drawings which illustrate a preferred, non-limiting example embodiment, and in which:

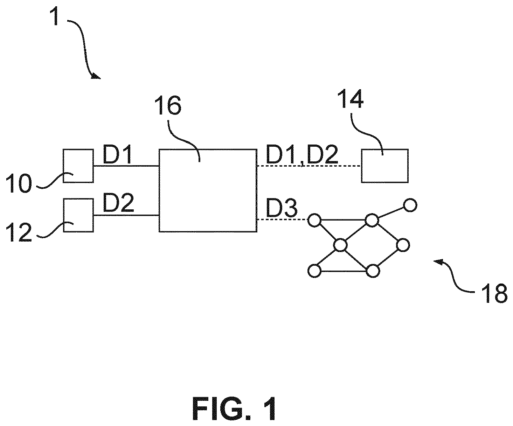

- Figure 1 shows a block diagram representing a health supervision system according to one or more embodiments.

Figure 1 shows a health supervision system 1 according to one or more embodiments. The system 1 allows setting up a company checkpoint where users, for example, employees, visitors and members of their families, can undergo epidemiological screening on a voluntary basis. Test results, for example, in the form of second data items D2, can be recorded in the local data repository 16, for example, a company database, and can be certified through blockchain technology as described in more detail below. Advantageously, it is thus possible to periodically monitor the state of health of a group of users in order to provide better protection for the company. For example, the user can periodically interact with the system 1 to undergo the health check-up. For example, each user can interact with the system 1 at predetermined regular intervals, for example, weekly or twice weekly.

Advantageously, converting a company into a health centre to guarantee the health of employees and members of their families can make it easier for shops and restaurants and other trading enterprises to reopen safely, thus allowing a safe and effective transition out of the emergency situation.

The system 1 comprises:
- an identification station 10, configured to obtain at least one first data item D1 indicating an identity of a user,
- a health station 12 configured to obtain at least one second data item D2 indicating a health situation of the user identified,
- at least one local data repository 14, and
- a control unit 16, configured for:
   - receiving the at least one first data item D1 and the at least one second data item D2,
   - calculating a string D3, as a function of the at least one first data item D1 and/or of the at least one second data item D2, using a cryptographic function, where the string D3 indicates, for example, the first data item D1 and/or the second data item D2,
   - storing the at least one first data item D1 and/or the at least one second data item D2 in the at least one local data repository 14, and
   - transmitting the string D3 to a distributed architecture database 18 of the distributed ledger type to write the string D3 to the distributed architecture database, for example, a blockchain-based database.

According to an aspect, the system 1 may comprise the distributed architecture database 18 of the distributed ledger type. For example, the database 18 may be defined by a peer-to-peer network based on blockchain technology. The database 18 may be defined by a plurality of nodes within one or more companies forming part of a health supervision network, and/or within local health care authorities (ASL). According to an aspect, the control unit 16 may be configured to write the string D3 to the distributed architecture database 18.

Advantageously, this system allows ensuring the immutability of the data, in that it is based on the use of the distributed architecture database 18, while at the same time guaranteeing the users' right to be forgotten, in that the at least one first data item D1 and the at least one second data item D2 are not stored in the database 18. Instead, the string D3, indicating the data items D1 and D2, is stored in the database 18. In effect, if the results of an employee's swab test were stored in a blockchain, that information would become immutable (tamper proof) but would be visible to one or more organizations forming part of the same blockchain, in contrast with the right to personal data privacy, whose observance is even more stringent in the case of sensitive or health data.

In one or more embodiments, the identification station 10 may comprise a scanner, preferably a bar code scanner or a camera, configured to obtain the at least one first data item D1 comprising one or more elements identifying the user's identity. For example, the scanner may be configured to identify a user by means of an identification document such as, for example, an identity card, driver's licence and/or social security card. The scanner may thus capture the at least one first data item D1, which may include the name, surname and/or taxpayer ID number of a user. The identification station 10 may be connected directly or indirectly to the control unit 16 and may be configured to transmit the at least one first data item D1 to the control unit 16.

In one or more embodiments, the health station 12 may comprise a laboratory configured to perform a serological and/or molecular test on a biological sample extracted (taken) from the user identified and to obtain the at least one second data item D2 including a result of the serological and/or molecular test. The health station 12 may be connected directly or indirectly to the control unit 16 and may be configured to transmit the at least one second data item D2 to the control unit 16.

According to an aspect, the health supervision system 1 may comprise the offices of one or more companies. The identification station 10 may be located inside the offices of the one or more companies, each company having, associated with it, an identification station 10 and a health station 12. The health station 12 - for example, the laboratory - may be situated inside the offices of the one or more companies or it may be situated at a remote location, off company premises. In the latter case, after identification, the sample extracted (taken) from the user can be transmitted to the health station 12 for processing.

According to an aspect, the laboratory comprises highly qualified staff capable of working with the companies based on industrial methods and schedules.

According to an aspect, the health station 12 may be configured to perform a rapid serological test by analysing a blood sample using immuno-chromatography plates to detect IG-G and IG-M antibodies. The health station 12 may comprise a plurality of disposable kits capable of performing the serological test and providing a result in a relatively short space of time.

The epidemiological screening process might require that each person involved undergo a rapid serological test (subject to consent). In the event of a negative result (IG-M and IG-G negative), the test may be repeated within a predetermined time interval (for example, 15-20 days). If the rapid serological test gives a positive result (IG-M and/or IG-G), the screening process may involve a chemiluminescence analysis or ELISA. After the chemiluminescence test or ELISA, the screening process may comprise performing a molecular test to confirm the diagnosis for those who tested IG-M positive and IG-G positive or negative.

The chemiluminescence test or ELISA is a semi-quantitative in vitro assay for human antibodies, constituting cogent evidence to integrate the direct search for the pathogen. The instruments required include the specific approved kits as well as accessory instruments useful for performing the test. To obtain the result of the ELISA test, the laboratory may comprise basic laboratory instruments and consumables (calibrated pipettes, tips, chronometer, incubator and thermal bath, refrigerators) as well as instruments for reading the result (spectrophotometer).

The health station 12 may also be configured to carry out a molecular test based on RT-PCR methods to amplify the viral genes expressed during infection by SARS-CoV-2. In this case, the laboratory may also comprise instruments such as centrifuge, tweezers, a detector for nucleic acid (DNA/RNA) sample quality analysis and analysis of genes of pathogens derived from the human body by single fluorescence PCR.

The health station 12 may be divided into dedicated areas: for example, a first area for taking the sample; a second area for collecting the samples, in the case where the samples are processed off company premises; and lastly, a third laboratory area for analysis and final checking. If the health station 12 is totally in-house, the first, second and third areas may all be situated on company premises; alternatively, the third area, comprising the laboratory, may be situated at a location outside the company.

Advantageously, creating an in-house laboratory would allow reducing test times, through centralization of structures, attaining a high degree of innovation compared to the state of the art, with benefits for the entire production chain. Compared to using an external laboratory, an in-house laboratory would also be advantageous in economic and financial terms in the long term and considering the large number of tests to be carried out to keep employees and their families monitored. According to an aspect, the system 1 may require consent to process the data collected, that is, to process and record the at least one first data item D1 and the at least one second data item D2, whether the result is positive or negative. For example, storage may occur through use of a certified data repository.

As stated above, the system 1 can be subdivided into a network of companies, so as to be able to monitor a plurality of users. Each company can therefore record the first and second data items D1, D2 independently of each other, for example, in one or more local data repositories 14 and each company can record the data strings D3 in the same distributed architecture database 18 based on the same blockchain, for example, to ensure that all the data are recorded correctly and immutably.

Advantageously, when a relatively large number of companies forms part of the same blockchain, the health supervision system 1 can become tamper proof, or almost tamper proof, for example, even without the collaboration of local health authorities (ASL) or other public authorities.

According to an aspect, the at least one data repository 14 may be connected to the control unit 16, in hardwired or wireless manner, and may be configured to receive from it the at least one first and/or second data item D1, D2.

In one or more embodiments, the at least one local data repository 14 comprises a centralized data repository, the data repository being protected and accessible to the control unit 16 subject to authorization. For example, the first data items D1 and the second data items D2 may be accessed only through the control unit 16, for example, after entering a password. That way, access to the sensitive data is possible only if authorized.

For example, the first and second data items D1, D2 may be accessible only to public health and/or judicial organizations or to a company doctor.

In addition, or alternatively, the at least one local data repository 14 may be defined at least partly by a plurality of personal electronic devices, preferably personal smartphones. For example, a certain electronic device may be associated with each user. That way, the at least one second data item D2 of a certain identified user can be saved only to the personal electronic device of that user. In other words, the second data items D2 of individual users can be saved to different personal devices (a copy of the data items may be present in a centralized company database).

In addition, or alternatively, the at least one second data item D2 is accessible following a request from the user and/or from the control unit 16. Advantageously, the user can access their personal data even outside the context of the company, for example, if they are required to show they are negative to the virus in other circumstances, such as at a cinema, restaurant, stadium, and so on. At the same time, the control unit 16 can access the data present in the personal electronic devices, so that public health and/or judicial organizations can obtain the first and second data items D1, D2 of each user in the event of need.

In addition, or alternatively, the at least one second data item D2 can only be deleted following a command from the control unit 16. Advantageously, that way, the user cannot delete the second data items D2.

The second data items D2 in the personal electronic devices may be accessible to the user through a mobile application, hereinafter called "app".

The use of an app containing the results of tests may have one or more advantages: for example, the user can show the test results through the app in order to prove the absence of infection at the date of the test, so as to allow public commercial establishments to interact with their customers in a safe environment.

That way, at least part of the local data repository 14 might be resident in personal electronic devices.

According to an aspect, the database 18 may be connected to the control unit 16, in hardwired or wireless manner, and may be configured to receive from it the string D3.

In one or more embodiments, the distributed architecture database 18 of distributed ledger type is of the blockchain type and comprises information grouped in a plurality of information blocks interconnected by cryptographic algorithms.

According to an aspect, creating a blockchain shared by different companies in a certain geographical area may have one or more advantages: for example, it allows certifying the immutability of the first and second data items D1, D2 recorded by each individual company. For example, every company may record the data D1, D2 independently of the others in local data repositories 14 and, at the same, may record strings D3, indicating the data D1, D2 in the same blockchain, so as to ensure that all the data are recorded correctly and immutably.

Advantageously, when a sufficiently large number of different companies record their data on the blockchain, it will be easier to guarantee a tamper-proof system.

According to an aspect, the distributed architecture database 18 of the distributed ledger type may be defined by or built on the Ethereum or Ethereum Classic or Iota or Eos or NEO or Waves or Qtum or NEM or Multiversum or R3 Corda or R3 Corda enterprise or Hyperledger or Ripple or Stellar platform.

According to an aspect, the distributed architecture database 18 of the distributed ledger type may operate with a consensus protocol for writing to the database 18 and selected from the following types:
- proof of work;
- proof of stake;
- Corda consensus protocols (configured to reach consensus on the specific "state object");
- CFT (Crash fault tolerant), preferably implemented with Kafka and/or Zookeeper;
- Solo;
- BFT (Byzantine fault tolerance);
- PBFT (practical BFT);
- SBFT (simplified BFT);
- Raft;
- Sumeragi;
- PoET (proof of Elapsed Time);
- Permissioned Voting-based.

As stated earlier herein, the database 18 may be defined by a plurality of processing nodes. Each company may be associated with a specific node which is the first to check and consent to the addition of strings D3 to the distributed architecture database 18 of the distributed ledger type.

According to an aspect, the processing nodes may comprise a first set of processors which are configured to keep a complete copy of the distributed architecture database 18 and a second set of processors which are configured to keep a partial copy of the distributed architecture database 18. In this way, advantageously, the processing nodes of the second set can be simpler than those of the first set and integrate a reduced capacity memory.

Each string D3 has a time stamp associated with it. Preferably, the strings D3 may be interconnected with each other by cryptographic algorithms.

In one or more embodiments, the control unit 16 is therefore configured to record, retrieve and/or delete the first and second data items D1, D2 in the at least one local data repository 14. At the same time, the control unit 16 is configured to save the string D3 indicating the first and/or second data items D1, D2 to the database 18.

The string D3 may indicate the data items D1, D2 but at the same time it can protect the user's privacy in that the data items D1, D2 are not visible in unencrypted form. In other words, the string D3 can be calculated cryptographically.

The step of calculating the string with a cryptographic function may comprise calculating the string D3 with a cryptographic hash function, for example, with a secure hash algorithm SHA-256.

In one or more embodiments:
- the calculated string D3 has a biunique match with the at least one first data item D1 and/or the at least one second data item D2 from which the string D3 is calculated, and/or
- the at least one first and/or second data item D1, D2 cannot be obtained from the string D3.

That way, the test results - that is, the unencrypted second health data items D2 - can be saved in the local data repository 14, where the accessibility of the data repository 14 is subject to authorization. On the contrary, the strings D3, indicating at least the second data items D2 from which they are calculated, are freely accessible by anyone who has access to the database 18 so as to allow the data to be certified. In effect, the truthfulness of the data provided is guaranteed by the fact that the secure hash of these results has been saved in the blockchain.

In one or more embodiments, the control unit 16 is configured for:
- associating a time stamp with the at least one first data item D1 and/or with the at least one second data item D2, and
- deleting data - automatically, for example - between a plurality of first data items D1 and/or second data items D2 stored in the at least one local data repository 14 if the time stamp indicates that the data were stored before a certain date - for example, if the data were saved on or before a date six months previously.

Advantageously, this allows protecting the privacy and the right to be forgotten of users subjected to health supervision, in compliance with Italian and European regulations and with the recommendations of the data protection authority. In effect, the data recorded are not published in unencrypted form in the database 18 and can be deleted after a predetermined time interval which is, for example, established in agreement with the authorities. Upon the lapse of that time period, the system 1 automatically removes the obsolete data.

In one or more embodiments, the control unit 16 may be configured to transmit an alarm signal if the at least one second data item D2 indicates the presence of SARS-CoV-2 virus antigens, that is to say, if the user might be a carrier of serious acute respiratory syndrome coronavirus 2 or might exhibit symptoms of Covid-19.

According to an aspect, the alarm may be displayed on the personal electronic device of the user who has tested positive to the virus; in addition, or alternatively, the alarm may be transmitted to a company officer, for example, the doctor, to allow starting a procedure to contain the spread of infection.

One or more embodiments address a health supervision method, comprising the following steps:
- providing a health supervision system 1 according to one or more embodiments,
- obtaining with the identification station 10 at least one first data item D1 indicating the identity of a user,
- obtaining with the health station 12 at least one second data item D2 indicating the health situation of the user identified,
- receiving with the identification station 10 and the health station 12 the at least one first data item D1 and the at least one second data item D2,
- calculating a string D3, using a cryptographic function, as a function of the at least one first data item D1 and/or the at least one second data item D2,
- storing the at least one first data item D1 and/or the at least one second data item D2 in the at least one local data repository 14, and
- transmitting the string D3 to a distributed architecture database 18 of the distributed ledger type to write the string D3 to the distributed architecture database 18.

According to an aspect, the supervision method may comprise writing the string D3 to the database 18.

According to another aspect, the method can comprise the following steps:
- identifying a user;
- reading the distributed architecture database 18 and the local data repository 14 to retrieve information associated with the user;
- issuing an alarm signal if the reading of the distributed architecture database 18 in combination with the local data repository 14 gives as its result an anomalous health condition associated with the user. The scope of protection sought for is defined by the appended claims.

## Claims

1. A health supervision system (1), comprising:
- an identification station (10), configured to obtain at least one first data item (D1) indicating an identity of a user,
- a health station (12) configured to obtain at least one second data item (D2) indicating a health situation of the user identified,
- at least one local data repository (14), and
- a control unit (16), configured for:
- receiving the at least one first data item (D1) and the at least one second data item (D2),
- storing the at least one first data item (D1) and/or the at least one second data item (D2) in the at least one local data repository (14), wherein the health supervision system (1) is **characterized by** the fact that the control unit (16) is further configured for:
- calculating a string (D3), using a cryptographic function, as a function of the at least one first data item (D1) and/or the at least one second data item (D2), wherein:
- each string (D3) has a time stamp associated with it,
- the string (D3) is biuniquely matched with the at least one first data item (D1) and/or the at least one second data item (D2) from which the string (D3) is calculated, and/or
- the at least one first data item (D1) and/or the at least one second data item (D2) cannot be obtained from the string (D3),
- transmitting the string (D3) to a distributed architecture database (18) of the distributed ledger type to write the string (D3) to the distributed architecture database (18).

2. The supervision system (1) according to claim 1, wherein the identification station (12) comprises a scanner, preferably a camera or a bar code scanner, configured to obtain the at least one first data item (D1) comprising one or more elements identifying the user's identity.

3. The supervision system (1) according to claim 1 or 2, wherein the health station (12) comprises a laboratory configured to perform a serological and/or molecular test on a sample from the user and to obtain the at least one second data item (D2) comprising a result of the serological and/or molecular test.

4. The supervision system (1) according to any one of claims 1 to 3, wherein the at least one local data repository (14) comprises a centralized data repository, the data repository being protected and accessible to the control unit (16) following an authorization.

5. The supervision system (1) according to any one of the preceding claims, wherein the at least local data repository (14) is defined at least partly by a plurality of personal electronic devices, where each of the personal electronic devices is associated with a certain user, so that:
- the at least one second data item (D2) can be stored only in the personal electronic device of the user identified, and/or
- the at least one second data item (D2) is accessible following a request from the user and/or from the control unit (16), and/or
- the at least one second data item (D2) can only be deleted following a command from the control unit (16).

6. The supervision system (1) according to any one of the preceding claims, comprising the distributed architecture database (18) of distributed ledger type.

7. The supervision system (1) according to the preceding claim, wherein the distributed architecture database (18) of distributed ledger type is of the blockchain type and comprises information grouped in a plurality of information blocks interconnected by cryptographic algorithms.

8. The supervision system (1) according to claim 6 or 7, wherein the distributed architecture database (18) of the distributed ledger type is built on the Ethereum or Ethereum Classic or Iota or Eos or NEO or Waves or Qtum or NEM or Multiversum or R3 Corda or R3 Corda enterprise or Hyperledger or Ripple or Stellar platform.

9. The supervision system (1) according to any one of the preceding claims, wherein the distributed architecture database (18) of the distributed ledger type operates with a consensus protocol for writing to the database (18) and selected from the following types:
- proof of work;
- proof of stake;
- Corda consensus protocols (configured to reach consensus on the specific "state object");
- CFT (Crash fault tolerant), preferably implemented with Kafka and/or Zookeeper;
- Solo;
- BFT (Byzantine fault tolerance);
- PBFT (practical BFT);
- SBFT (simplified BFT);
- Raft;
- Sumeragi;
- PoET (proof of Elapsed Time);
- Permissioned Voting-based.

10. The health supervision system (1) according to any one of the preceding claims, wherein the step of calculating the string (D3) comprises calculating the string (D3) using a cryptographic hash function.

11. The health supervision system (1) according to any one of the preceding claims, wherein the control unit (16) is configured for:
associating a time stamp with the at least one first data item (D1) and/or with the at least one second data item (D2), and
deleting data between a plurality of first data items (D1) and/or second data items (D2) stored in the at least one local data repository (14) if the time stamp indicates that the data was stored before a certain date.

12. The health supervision system (1) according to any one of the preceding claims, wherein the control unit (16) is configured for transmitting an alarm signal if the at least one second data item (D2) indicates the presence of SARS-CoV-2 virus antigens.

13. A health supervision method for use in a health supervision system (1) according to any one of the preceding claims, comprising the steps:
- obtaining with the identification station (10) at least one first data item (D1) indicating the identity of a user,
- obtaining with the health station (12) at least one second data item (D2) indicating the health situation of the user identified,
- receiving with the identification station (10) and the health station (12) the at least one first data item (D1) and the at least one second data item (D2),
- storing the at least one first data item (D1) and/or the at least one second data item (D2) in the at least one local data repository (14), the method further comprising the following steps:
- calculating a string (D3), using a cryptographic function, as a function of the at least one first data item (D1) and/or the at least one second data item (D2), the string (D3) being biuniquely matched with the at least one first data item (D1) and/or the at least one second data item (D2) from which the string (D3) is calculated, and/or the at least one first data item (D1) and/or the at least one second data item (D2) being not obtainable from the string (D3),- associating a time stamp with the string (D3),
- transmitting the string (D3) to a distributed architecture database (18) of the distributed ledger type to write the string (D3) to the distributed architecture database (18).

## Patentansprüche

1. Gesundheitskontrollsystem (1), umfassend:
- eine Identifikationsstation (10), die ausgelegt ist, um mindestens ein erstes Datenelement (D1) zu erhalten, das eine Identität eines Benutzers anzeigt,
- eine Gesundheitsstation (12), die ausgelegt ist, um mindestens ein zweites Datenelement (D2) zu erhalten, das eine Gesundheitssituation des identifizierten Benutzers anzeigt,
- mindestens ein lokales Daten-Repository (14) und
- eine Steuereinheit (16), ausgelegt zum:
- Empfangen des mindestens einen ersten Datenelements (D1) und des mindestens einen zweiten Datenelements (D2),
- Speichern des mindestens einen ersten Datenelements (D1) und/oder des mindestens einen zweiten Datenelements (D2) in dem mindestens einen lokalen Daten-Repository (14),
wobei das Gesundheitskontrollsystem (1) **dadurch gekennzeichnet ist, dass** die Steuereinheit (16) ferner ausgelegt ist, zum:
- Berechnen einer Zeichenfolge (D3) unter Verwendung einer kryptographischen Funktion als Funktion des mindestens einen ersten Datenelements (D1) und/oder des mindestens einen zweiten Datenelements (D2), wobei:
- eine jede Zeichenfolge (D3) einen Zeitstempel aufweist, der ihr zugeordnet ist,
- die Zeichenfolge (D3) mit dem mindestens einen ersten Datenelement (D1) und/oder dem mindestens einen zweiten Datenelement (D2), aus dem die Zeichenfolge (D3) berechnet wird, bi-eindeutig übereinstimmt und/oder
- das mindestens eine erste Datenelement (D1) und/oder das mindestens eine zweite Datenelement (D2) aus der Zeichenfolge (D3) nicht erhalten werden kann,
- Übertragen der Zeichenfolge (D3) an eine verteilte Architekturdatenbank (18) vom verteilten Ledger-Typ, um die Zeichenfolge (D3) in die verteilte Architekturdatenbank (18) zu schreiben.

2. Kontrollsystem (1) nach Anspruch 1, wobei die Identifikationsstation (12) einen Scanner, vorzugsweise eine Kamera oder einen Barcode-Scanner, umfasst, der ausgelegt ist, um das mindestens eine erste Datenelement (D1) zu erhalten, das ein oder mehrere Elemente umfasst, die die Identität des Benutzers identifizieren.

3. Kontrollsystem (1) nach Anspruch 1 oder 2, wobei die Gesundheitsstation (12) ein Labor umfasst, das dazu ausgelegt ist, einen serologischen und/oder molekularen Test an einer Probe des Benutzers durchzuführen und das mindestens eine zweite Datenelement (D2) zu erhalten, das ein Ergebnis des serologischen und/oder molekularen Tests umfasst.

4. Kontrollsystem (1) nach einem der Ansprüche 1 bis 3, wobei das mindestens eine lokale Daten-Repository (14) ein zentralisiertes Daten-Repository umfasst, wobei das Daten-Repository geschützt und nach einer Autorisierung für die Steuereinheit (16) zugänglich ist.

5. Kontrollsystem (1) nach einem der vorhergehenden Ansprüche, wobei das mindestens eine lokale Daten-Repository (14) zumindest teilweise durch eine Vielzahl von persönlichen elektronischen Vorrichtungen definiert ist, wobei eine jede der persönlichen elektronischen Vorrichtungen einem bestimmten Benutzer zugeordnet ist, sodass:
- das mindestens eine zweite Datenelement (D2) nur in der persönlichen elektronischen Vorrichtung des identifizierten Benutzers gespeichert werden kann, und/oder
- auf das mindestens eine zweite Datenelement (D2) auf Anforderung des Benutzers und/oder der Steuereinheit (16) zugegriffen werden kann, und/oder
- das mindestens eine zweite Datenelement (D2) nur nach einem Befehl von der Steuereinheit (16) gelöscht werden kann.

6. Kontrollsystem (1) nach einem der vorhergehenden Ansprüche, umfassend die verteilte Architekturdatenbank (18) vom verteilten Ledger-Typ.

7. Kontrollsystem (1) nach dem vorhergehenden Anspruch, wobei die verteilte Architekturdatenbank (18) vom verteilten Ledger-Typ vom Blockchain-Typ ist und Informationen umfasst, die in einer Vielzahl von Informationsblöcken gruppiert sind, die durch kryptografische Algorithmen miteinander verbunden sind.

8. Kontrollsystem (1) nach Anspruch 6 oder 7, wobei die verteilte Architekturdatenbank (18) vom verteilten Ledger-Typ auf der Ethereum- oder Ethereum Classic- oder Iota- oder Eos- oder NEO- oder Waves- oder Qtum- oder NEM- oder Multiversum- oder R3 Corda- oder R3 Corda Enterprise- oder Hyperledger- oder Ripple- oder Stellar-Plattform aufgebaut ist.

9. Kontrollsystem (1) nach einem der vorhergehenden Ansprüche, wobei die verteilte Architekturdatenbank (18) vom verteilten Ledger-Typ mit einem Konsensprotokoll zum Schreiben in die Datenbank (18) arbeitet, das aus den folgenden Typen ausgewählt ist:
- Nachweis der Arbeit;
- Nachweis des Einsatzes;
- Corda-Konsensprotokolle (ausgelegt, um einen Konsens über das spezifische "Zustandsobjekt" zu erreichen);
- CFT (Crash Fault Tolerant), vorzugsweise implementiert mit Kafka und/oder Zookeeper;
- Solo;
- BFT (Byzantine Fault Tolerance);
- PBFT (practical BFT);
- SBFT (simplified BFT);
- Raft;
- Sumeragi;
- PoET (Proof of Elapsed Time);
- Permissioned Voting-basiert.

10. Gesundheitskontrollsystem (1) nach einem der vorhergehenden Ansprüche, wobei der Schritt zum Berechnen der Zeichenfolge (D3) das Berechnen der Zeichenfolge (D3) unter Verwendung einer kryptografischen Hash-Funktion umfasst.

11. Gesundheitskontrollsystem (1) nach einem der vorhergehenden Ansprüche, wobei die Steuereinheit (16) ausgelegt ist, zum:
Zuordnen eines Zeitstempels zu dem zumindest einen ersten Datenelement (D1) und/oder zu dem zumindest einen zweiten Datenelement (D2), und
Löschen von Daten zwischen einer Vielzahl von in dem zumindest einen lokalen Daten-Repository (14) gespeicherten ersten Datenelementen (D1) und/oder zweiten Datenelementen (D2), wenn der Zeitstempel anzeigt, dass die Daten vor einem bestimmten Datum gespeichert wurden.

12. Gesundheitskontrollsystem (1) nach einem der vorhergehenden Ansprüche, wobei die Steuereinheit (16) zum Übertragen eines Alarmsignals ausgelegt ist, wenn das mindestens eine zweite Datenelement (D2) das Vorhandensein von SARS-CoV-2-Virusantigenen anzeigt.

13. Gesundheitskontrollverfahren zur Verwendung in einem Gesundheitskontrollsystem (1) nach einem der vorhergehenden Ansprüche, umfassend die Schritte:
- Erhalten mit der Identifikationsstation (10) mindestens eines ersten Datenelements (D1), das die Identität eines Benutzers anzeigt,
- Erhalten mit der Gesundheitsstation (12) mindestens eines zweiten Datenelements (D2), das die Gesundheitssituation des identifizierten Benutzers anzeigt,
- Empfangen mit der Identifikationsstation (10) und der Gesundheitsstation (12) des mindestens einen ersten Datenelements (D1) und des mindestens einen zweiten Datenelements (D2),
- Speichern des mindestens einen ersten Datenelements (D1) und/oder des mindestens einen zweiten Datenelements (D2) in dem mindestens einen lokalen Daten-Repository (14),
wobei das Verfahren ferner die folgenden Schritte umfasst:
- Berechnen einer Zeichenfolge (D3) unter Verwendung einer kryptographischen Funktion als Funktion des mindestens einen ersten Datenelements (D1) und/oder des mindestens einen zweiten Datenelements (D2), wobei die Zeichenfolge (D3) mit dem mindestens einen ersten Datenelement (D1) und/oder dem mindestens einen zweiten Datenelement (D2), aus dem die Zeichenfolge (D3) berechnet wird, bi-eindeutig übereinstimmt und/oder wobei das mindestens eine erste Datenelement (D1) und/oder das mindestens eine zweite Datenelement (D2) aus der Zeichenfolge (D3) nicht erhältlich ist,
- Zuordnen eines Zeitstempels zu der Zeichenfolge (D3),
- Übertragen der Zeichenfolge (D3) an eine verteilte Architekturdatenbank (18) vom verteilten Ledger-Typ, um die Zeichenfolge (D3) in die verteilte Architekturdatenbank (18) zu schreiben.

## Revendications

1. Système de supervision de la santé (1), comprenant :
- un poste d'identification (10), configuré pour obtenir au moins un premier élément de données (D1) indiquant une identité d'un utilisateur,
- un poste de santé (12), configuré pour obtenir au moins un second élément de données (D2) indiquant un état de santé de l'utilisateur identifié,
- au moins un référentiel de données (14) local, et
- une unité de contrôle (16), configurée pour :
- recevoir l'au moins un premier élément de données (D1) et l'au moins un second élément de données (D2),
- stocker l'au moins un premier élément de données (D1) et/ou l'au moins un second élément de données (D2) dans l'au moins un référentiel de données (14) local,
dans lequel le système de supervision de la santé (1) est **caractérisé en ce que** l'unité de contrôle (16) est en outre configurée pour :
- calculer une chaîne (D3), à l'aide d'une fonction cryptographique, en fonction de l'au moins un premier élément de données (D1) et/ou de l'au moins un second élément de données (D2), dans lequel :
- chaque chaîne (D3) est associée à un horodatage,
- la chaîne (D3) est mise en correspondance de manière biunivoque avec l'au moins un premier élément de données (D1) et/ou l'au moins un second élément de données (D2) à partir desquels la chaîne (D3) est calculée, et/ou
- l'au moins un premier élément de données (D1) et/ou l'au moins un second élément de données (D2) ne peuvent être obtenus à partir de la chaîne (D3),
- transmettre la chaîne (D3) à une base de données (18) à architecture distribuée du type registre distribué pour écrire la chaîne (D3) dans la base de données (18) à architecture distribuée.

2. Système de supervision (1) selon la revendication 1, dans lequel le poste d'identification (12) comprend un scanner, de préférence une caméra ou un lecteur de codes-barres, configuré pour obtenir l'au moins un premier élément de données (D1) comprenant un ou plusieurs éléments identifiant l'identité de l'utilisateur.

3. Système de supervision (1) selon la revendication 1 ou 2, dans lequel le poste de santé (12) comprend un laboratoire configuré pour réaliser un test sérologique et/ou moléculaire sur un échantillon provenant de l'utilisateur et pour obtenir l'au moins un second élément de données (D2) comprenant un résultat du test sérologique et/ou moléculaire.

4. Système de supervision (1) selon l'une quelconque des revendications 1 à 3, dans lequel l'au moins un référentiel de données (14) local comprend un référentiel de données centralisé, le référentiel de données étant protégé et accessible à l'unité de contrôle (16) suite à une autorisation.

5. Système de supervision (1) selon l'une quelconque des revendications précédentes, dans lequel l'au moins un référentiel de données (14) local est défini au moins en partie par une pluralité de dispositifs électroniques personnels, où chacun des dispositifs électroniques personnels est associé à un certain utilisateur, de sorte que :
- l'au moins un second élément de données (D2) ne peut être stocké que dans le dispositif électronique personnel de l'utilisateur identifié, et/ou
- l'au moins un second élément de données (D2) soit accessible à une demande de l'utilisateur et/ou de l'unité de contrôle (16), et/ou
- l'au moins un second élément de données (D2) ne peut être supprimé qu'à la suite d'un contrôle de l'unité de contrôle (16).

6. Système de supervision (1) selon l'une quelconque des revendications précédentes, comprenant la base de données (18) à architecture distribuée de type registre distribué.

7. Système de supervision (1) selon la revendication précédente, dans lequel la base de données (18) à architecture distribuée de type registre distribué est de type à chaîne de blocs et comprend des informations regroupées dans une pluralité de blocs d'information interconnectés par des algorithmes cryptographiques.

8. Système de supervision (1) selon la revendication 6 ou 7, dans lequel la base de données (18) à architecture distribuée de type registre distribué est construite sur la plateforme Ethereum ou Ethereum Classic ou Iota ou Eos ou NEO ou Waves ou Qtum ou NEM ou Multiversum ou R3 Corda ou R3 Corda enterprise ou Hyperledger ou Ripple ou Stellar.

9. Système de supervision (1) selon l'une quelconque des revendications précédentes, dans lequel la base de données (18) à architecture distribuée de type registre distribué fonctionne avec un protocole de consensus pour l'écriture dans la base de données (18) qui est choisi parmi les types suivants :
- preuve de travail ;
- preuve d'enjeu ;
- protocoles de consensus Corda (configurés pour atteindre un consensus sur l'« objet d'état » spécifique) ;
- CFT (Crash fault tolerant), de préférence mis en œuvre avec Kafka et/ou Zookeeper ;
- Solo ;
- BFT (Byzantine fault tolerance) ;
- PBFT (practical BFT) ;
- SBFT (simplified BFT) ;
- Raft ;
- Sumeragi ;
- PoET (preuve du temps écoulé) ;
- basé sur le vote autorisé.

10. Système de supervision de la santé (1) selon l'une quelconque des revendications précédentes, dans lequel l'étape de calcul de la chaîne (D3) comprend le calcul de la chaîne (D3) à l'aide d'une fonction de hachage cryptographique.

11. Système de supervision de la santé (1) selon l'une quelconque des revendications précédentes, dans lequel l'unité de contrôle (16) est configurée pour :
associer un horodatage à l'au moins un premier élément de données (D1) et/ou à l'au moins un second élément de données (D2), et
supprimer des données entre une pluralité de premiers éléments de données (D1) et/ou de seconds éléments de données (D2) stockés dans l'au moins un référentiel de données (14) local si l'horodatage indique que les données ont été stockées avant une certaine date.

12. Système de supervision de la santé (1) selon l'une quelconque des revendications précédentes, dans lequel l'unité de contrôle (16) est configurée pour transmettre un signal d'alarme si l'au moins un second élément de données (D2) indique la présence d'antigènes du virus SARS-CoV-2.

13. Procédé de supervision de la santé à utiliser dans un système de supervision de la santé (1) selon l'une quelconque des revendications précédentes, comprenant les étapes consistant à :
- obtenir avec le poste d'identification (10) au moins un premier élément de données (D1) indiquant l'identité d'un utilisateur,
- obtenir avec le poste de santé (12) au moins un second élément de données (D2) indiquant l'état de santé de l'utilisateur identifié,
- recevoir du poste d'identification (10) et du poste de santé (12) l'au moins un premier élément de données (D1) et l'au moins un second élément de données (D2),
- stocker l'au moins un premier élément de données (D1) et/ou l'au moins un second élément de données (D2) dans l'au moins un référentiel de données (14) local,
le procédé comprenant en outre les étapes suivantes :
- calculer une chaîne (D3), à l'aide d'une fonction cryptographique, en fonction de l'au moins un premier élément de données (D1) et/ou de l'au moins un second élément de données (D2), la chaîne (D3) étant mise en correspondance de manière biunivoque avec l'au moins un premier élément de données (D1) et/ou l'au moins un second élément de données (D2) à partir desquels la chaîne (D3) est calculée, et/ou avec l'au moins un premier élément de données (D1) et/ou l'au moins un second élément de données (D2) ne pouvant être obtenus à partir de la chaîne (D3),
- associer un horodatage à la chaîne (D3),
- transmettre la chaîne (D3) à une base de données (18) à architecture distribuée du type registre distribué pour écrire la chaîne (D3) dans la base de données (18) à architecture distribuée.
